(19) Europäisches Patentamt
European Patent Office
Office européen des brevets

(11) Publication number: **0 270 378 A2**

# (12) EUROPEAN PATENT APPLICATION

(21) Application number: 87310712.2

(22) Date of filing: 04.12.87

(51) Int. Cl.[4]: C 07 D 241/44, C 07 D 253/08, C 07 D 213/64, C 07 D 277/68, C 07 D 263/58, A 01 N 43/60, A 01 N 43/707, A 01 N 43/40, A 01 N 43/76

(30) Priority: 05.12.86 US 938618 04.09.87 US 93489

(43) Date of publication of application: 08.06.88 Bulletin 88/23

(84) Designated Contracting States: ES

(71) Applicant: E.I. DU PONT DE NEMOURS AND COMPANY
1007 Market Street
Wilmington Delaware 19898 (US)

(72) Inventor: Fawzi, Maged Mohamed
17 Beech Hill Drive
Newark Delaware 19711 (US)

(74) Representative: Hildyard, Edward Martin et al
Frank B. Dehn & Co. Imperial House 15-19 Kingsway
London WC2B 6UZ (GB)

## (54) Herbicidal aryloxybenzeneacetic acid derivatives.

(57) Compounds of Formula II

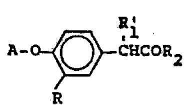

II

where

A is 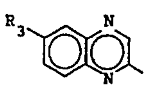 , 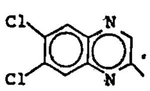 ,

A-1 A-2

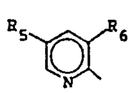 , 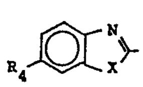 or 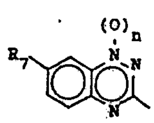 ;

A-4 A-5 A-6

X is O or S;
n is 0 or 1;
R is H, Cl, F, Br, $CH_3$, $OCH_3$ or $OCH_2CH_3$;
$R_1'$ is $C_1$-$C_3$ alkyl, allyl or propargyl;
$R_2$ is $X_1R_8$ or OH;
$R_3$ is Cl, F, Br or $CF_3$;
$R_4$ is H, Cl, F or Br;
$R_5$ is $CF_3$;
$R_6$ is H or Cl;
$R_7$ is H, Cl, F, Br or $CF_3$;
$R_8$ is an organic group of defined structure;
$X_1$ is O or S;
and their agriculturally suitable salts, exhibit herbicidal activity, especially for the control of weeds in rice.

EP 0 270 378 A2

## HERBICIDAL ARYLOXYBENZENEACETIC ACID DERIVATIVES

### Background of the Invention

This invention relates to herbicidally active phenylacetic acid derivatives, agriculturally suitable compositions thereof and a method for their use as herbicides.

The presence of undesirable vegetation causes substantial damage to useful crops, especially agricultural products that satisfy man's basic food and fiber needs, such as cotton, soybeans, rice and the like. The current population explosion and concomitant world food and fiber shortage underlie the need for improvements in the efficiency of producing these crops. Preventing or minimizing the loss of a portion of such valuable crops by killing or inhibiting the growth of undesired vegetation is one way of improving this efficiency.

A wide variety of materials useful for killing or inhibiting (controlling) the growth of undesired vegetation is available; such materials are commonly referred to as herbicides. The need exists, however, for still more effective herbicides that destroy or control weeds without causing significant damage to useful crops.

DE-A-30 04 770, which was published on August 28, 1980, discloses quinoxaline and quinoline compounds of the formula:

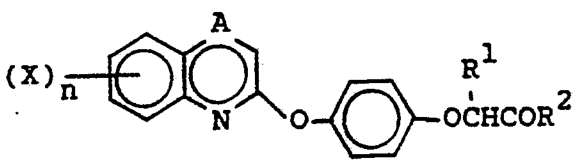

wherein

A is CH or N;

X is halogen;

n is 0, 1 or 2;

$R^1$ is H or lower alkyl;

$R^2$ is OH, alkoxy, OM, $NR^3R^4$, lower alkenyloxy, benzyloxy, alkoxyalkoxy, phenoxy, cyclohexyloxy, haloalkoxy, alkynyloxy or cyanoalkoxy;

M is an organic or inorganic cation;

$R^3$ is H or lower alkyl; and

$R_4$ is H or lower alkyl.

EP-A-23,785, published on February 11, 1981, discloses herbicidal quinoxaline compounds of the general formula:

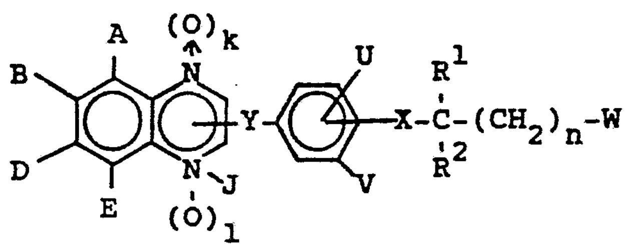

where

A, B, D, E, J, U and V may be halogen, among others;

$R^1$ may be H, $C_1$-$C_6$ alkyl, $C_2$-$C_6$ alkenyl, $C_2$-$C_6$ alkoxyalkyl, $C_1$-$C_6$ haloalkyl, acetyl, propionyl or $C_2$-$C_6$ alkoxycarbonyl;

$R^2$ may be H, $C_1$-$C_6$ alkyl, $C_2$-$C_6$ alkenyl, $C_2$-$C_6$ alkoxyalkyl, $C_1$-$C_6$ haloalkyl, or $R^1$ and $R^2$ taken together may form a methylene, ethylidene, propylidene or isopropylidene group; and

W may be COG and G may be $C_1$-$C_{10}$ alkoxy, $C_2$-$C_{10}$ alkenyloxy, and others.

EP-A-42,750, Published on December 30, 1981, discloses herbicidal quinoxaline compounds of general formula:

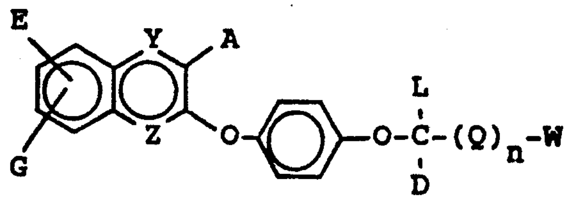

(I)

where

A is H or $C_1$-$C_4$ alkyl;
E is H, Cl, Br, F, $CF_3$, $CH_3$ or $OCH_3$;
G is H or Cl;
D is H, $CH_3$ or $CO_2R_8$;
L is H, $CH_3$ or $C_2H_5$;
Q is -CH=CH- or $-CH_2CH_2-$;
n is 0 or 1;
W is CN or C(O) or

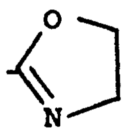 ;

and

Y and Z are independently N or N--O.

U.S. Patent 4,236,912, issued to Johnston et al. on December 2, 1980, discloses herbicidal quinolinyloxyphenoxy ethers of the formula

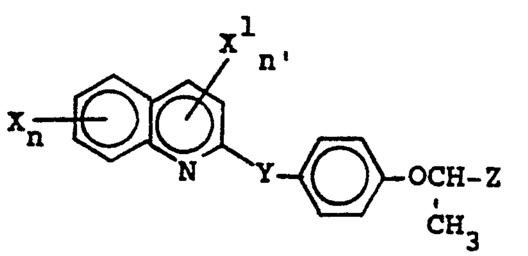

where

X and $X^1$ are independently F, Cl, Br, $CF_3$, $OCH_3$, $NO_2$ or $-N(R^1)_2$, provided that both cannot be $CF_3$, $OCH_3$, $NO_2$ or $-N(R^1)_2$;
n is 0, 1 or 2;
n′ is 0 or 1;
Y is O or S; and
Z is $-CO_2H$, $-CO_2M$, $-CO_2R$, -COSR, $-CONR^1_2$, $-CSNH_2$, CN, $-CH_2OR^1$ or $-CH_2O_2CR^1$.

Belgian Patent 858,618 discloses herbicidal compounds of the formula

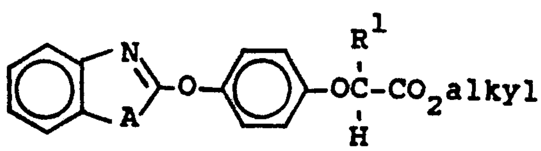

where

A = O, S, N-alkyl, and
$R^1$ = H or $C_1$-$C_4$ alkyl.

U.S. Patent 4,046,553, issued to Takahashi et al. on September 6, 1977, discloses herbicidal compounds of the formula

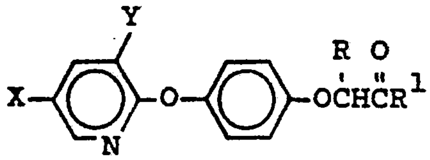

where

X is halogen;

Y is H, halogen or methyl;

R is H or $C_1$-$C_6$ alkyl; and

$R^1$ is hydroxy, $C_1$-$C_9$ alkoxy, $C_2$-$C_4$ alkenyloxy and others.

U.S. Patent 4,309,211, issued to Serban et al. on January 5, 1982, discloses herbicidal compounds of the formula

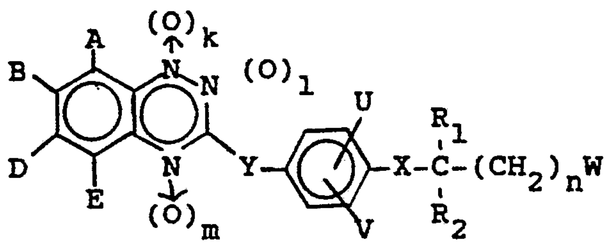

where

$R_1$ is H, alkyl, alkenyl, alkoxyalkyl, haloalkyl acetyl, propionyl or alkoxycarbonyl;

$R_2$ is H, alkyl, alkenyl, alkoxyalkyl or haloalkyl;

A, B, D and E may be halogen; and

W may be C(O)G where G is alkoxy.

Summary of the Invention

Novel compounds have been found of Formula II.

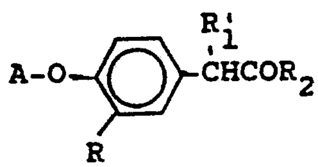

II

where

A is 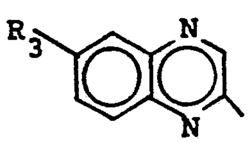 , 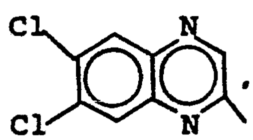 , ,

A-1 A-2

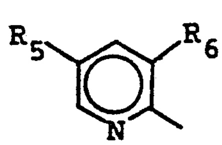 , 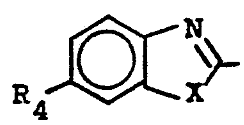 or 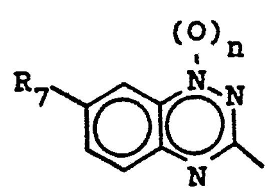 ;

A-4 A-5 A-6

X is O or S;

n is O or 1;
R is H, Cl, F, Br, $CH_3$, $OCH_3$ or $OCH_2CH_3$;
$R_1^{'}$ is $C_1$-$C_3$ alkyl, allyl or propargyl;
$R_2$ is $X_1R_8$ or OH;
$R_3$ is Cl, F, Br or $CF_3$;
$R_4$ is H, Cl, F or Br;
$R_5$ is $CF_3$;
$R_6$ is H or Cl;
$R_7$ is H, Cl, F, Br or $CF_3$;
$R_8$ is $C_1$-$C_4$ alkyl, benzyl, phenyl, $C_5$-$C_8$ cycloalkyl, $CH_2CH_2OCH_3$, $CH_2CH_2OCH_2CH_3$, $C_3$-$C_4$- alkenyl, $C_3$-$C_4$ alkynyl, $C_3$-$C_4$ alkenyl substituted with Cl, $CH_2CH_2OCH_2CH_2OCH_3$, $CH_2CH_2OCH_2CH_2OCH_2CH_3$, $CH_2CO_2CH_3$ or $CH(CH_3)CO_2CH_3$;
$X_1$ is O or S;

provided that for compounds of Formula II when A is A-2 or A-6, then R is H; and their agriculturally suitable salts.

In the above definitions, the term "alkyl" denotes straight chain or branched alkyl, e.g., methyl, ethyl, n-propyl, isopropyl or the different butyl isomers.

Alkenyl denotes straight chain or branched alkenes, e.g., 1-propenyl, 2-propenyl, 3-propenyl and the different butenyl isomers.

Alkynyl denotes straight chain or branched alkenyl, e.g., 2-propynyl and the different butynyl isomers.

Cycloalkyl denotes cyclopentyl, cyclohexyl, cycloheptyl and cyclooctyl.

The total number of carbon atoms in a substituent group Is indicated by the $C_i$-$C_j$ prefix where i and j are numbers from 1 to 8. For example, $C_1$-$C_4$ alkyl would designate methyl, ethyl, 1-propyl, 1-methylethyl and the various butyl isomers.

The preferred compounds of the invention for their higher herbicidal activity and/or more favorable ease of synthesis are:

1) Compounds of Formula II wherein $R_1^{'}$ is $CH_3$ and $R_2$ is $X_1R_8$.
2) Compounds of Preferred 1 wherein A is A-1, A-2, A-4, or A-5; R is H, Cl, $OCH_3$ or F; and $R_8$ is $C_1$-$C_4$ alkyl.
3) Compounds of Preferred 2 wherein A is A-1,
4) Compounds of Preferred 2 wherein A is A-2.
5) Compounds of Preferred 2 wherein A is A-4.
6) Compounds of Preferred 2 wherein A is A-5.
7) Compounds of Preferred 2 wherein A is A-1 and $R_8$ is $C_1$-$C_2$ alkyl.
8) Compounds of Preferred 2 wherein A is A-2 and $R_8$ is $C_1$-$C_2$ alkyl.
9) Compounds of Preferred 2 wherein A is A-4 and $R_8$ is $C_1$-$C_2$ alkyl.
10) Compounds of Preferred 2 wherein A is A-5 and $R_8$ is $C_1$-$C_2$ alkyl.

Compounds of the invention which are specifically preferred for reasons of greatest herbicidal activity and/or most favorable ease of synthesis are 3-chloro-4-(6-chloro-2-quinoxalinyloxy)-α-methylbenzeneacetic acid, methyl ester, m.p. 84-88°C; and 4-(3-chloro-5-(trifluoromethyl)-2-pyridinyloxy)α-methylbenzeneacetic acid, methyl ester, m.p. 46-48°C.

## DETAILED DESCRIPTION OF THE INVENTION

### Synthesis

I) Compounds of Formula II where $R_1^{'}$ is $CH_3$ or $C_2H_5$ and $R_2$ = $C_1$-$C_3$ alkoxy

The various compounds can be prepared by combining, preferably in equimolar amounts, the haloheterocycle and the alkali metal salt of the alkyl 4-hydroxy-α-substituted benzeneacetate as illustrated below using 2,6-dichloroquinoxaline as the haloheterocycle moiety.

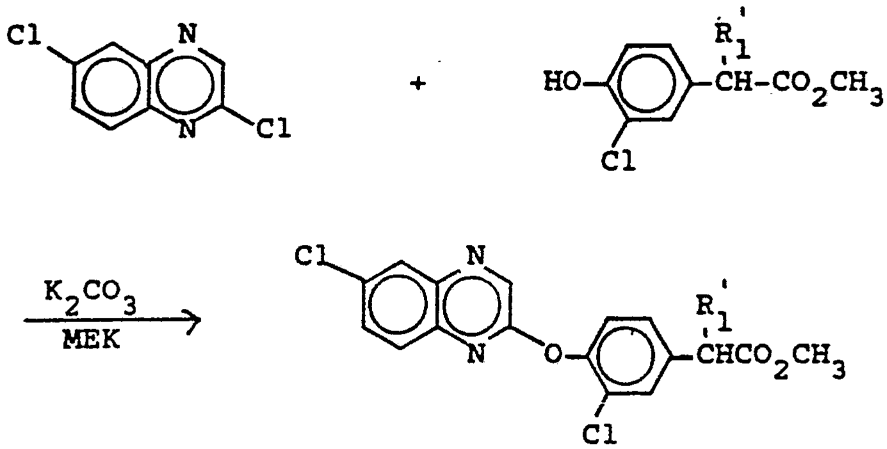


III

Suitable solvent for the reaction include dimethylformamide, dimethylsulfoxide, methyl ethyl ketone and acetonitrile. The reaction is preferably carried out at a temperature between 25 and 100°C.

II. Compounds of Formula II where $R_2$ = OH

Hydrolysis of compounds of Formula III where $R_2$ =$OC_1$-$C_3$ alkyl yields the acids. for example,

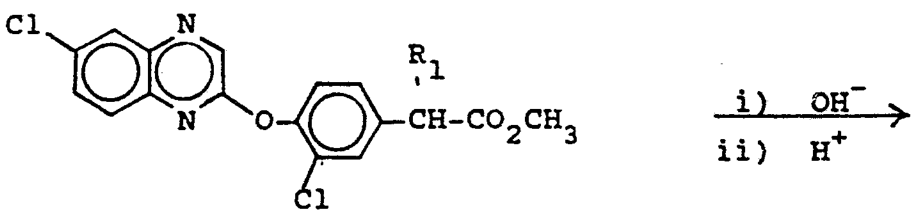


III

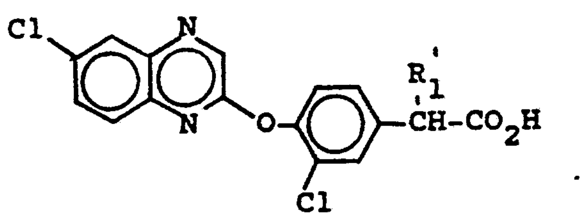

IV

III. Compounds of Formula II where $R_2$ = $X_1R_8$

The acids can be converted to the esters by standard synthetic procedures familiar to those skilled in the art. One of the methods that can be employed is outlined below:

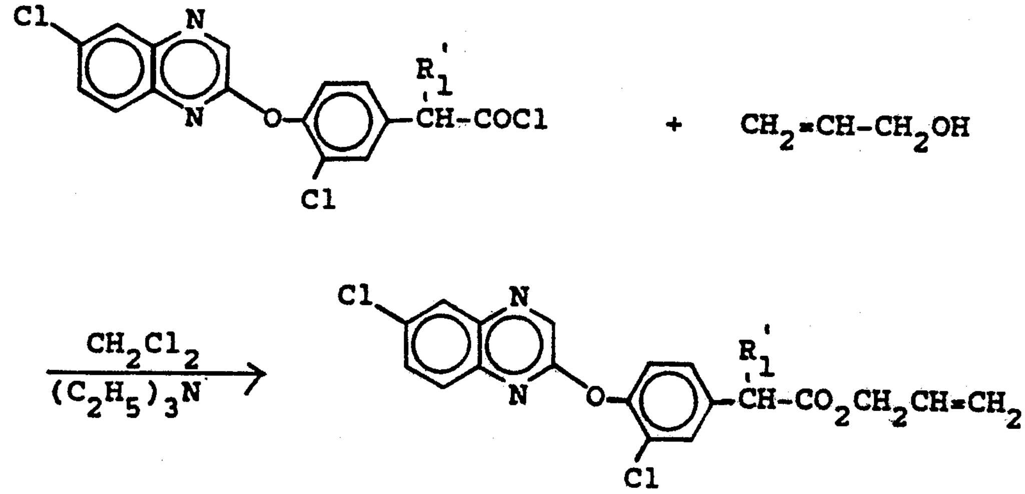


V

IV. Compounds of Formula II where $R_1' = CH_3$ or $C_2H_5$ and $R_2 = C_1$-$C_3$ alkoxy

The various compounds can be prepared by the interaction of the appropriate alkylphenylacetate with methyl or ethyl iodide in the presence of sodium hydride in dimethylformamide as shown below:

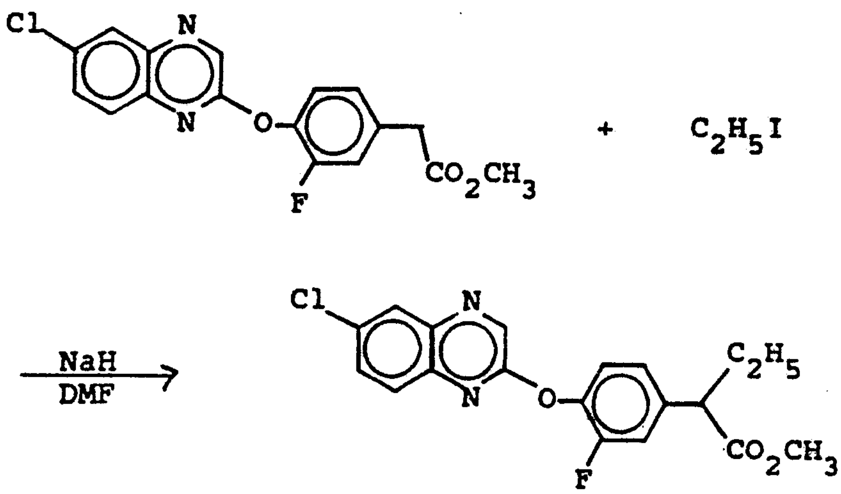


VI

The following Examples illustrate the preparation of some of the compounds of the invention.

Example 1

Methyl 4-[3-chloro-5-(trifluoromethyl)-2-pyridinyloxy]-α-methylbenzeneacetate

The title compound was prepared from 2,3-dichloro-5-trifluoromethylpyridine and methyl 4-hydroxy-α-methylbenzeneacetate by the procedure described under Example 1. The crude product was purified by flash chromatography (Silica Gel 60, 230-400 mesh ASTM, E. Merch) with n-chlorobutane as elution solvent, m.p. 46-48°C.

Anal. Calcd, for $C_{16}H_{13}ClF_3NO_3$: C, 53.41; H, 3.62; N, 3.89.

Found: C, 53.39; H, 3.70; N, 3.87.

Example 2

Methyl 4-(6,7-dichloro-2-quinoxalinyloxy)-α-methylbenzeneacetate

In a nitrogen atmosphere, 5 g (0.014 mol) methyl 4-(6,7-dichloro-2-quinoxalinyloxy)benzeneacetate was added at about 0° C to 2 g (0.14 mol) methyl iodide and a suspension of 0.7 g (.014 mol) 50% sodium hydride in 60 cc dimethylformamide. The reaction mixture was stirred and the temperature was allowed to rise to room temperature. After 5 hours, methanol (5 cc) was added and the reaction mixture was diluted with methylene chloride (200 cc), the resulting solution was washed with water (3 × 100 cc) and dried over magnesium sulfate. The methylene chloride was removed under vacuum and the crude product was crystallized once from butyl chloride-hexane and a second time from ethanol. yield 2 g, m.p. 117-120° C.

Anal. Calcd. for $C_{18}H_{14}Cl_2N_2O_3$: C, 57.29; H, 3.71; N, 7.43.

Found: C, 57.21; H, 3.67; N, 7.34.

Following the teachings of Examples 1 and 2 and by utilizing the appropriate 2-haloheterocycles and the appropriate alkyl α-methylbenzeneacetate or the alkyl halide and the alkyl substituted benzeneacetates the ethers of Formula II listed in the following Tables can be prepared.

## Table I

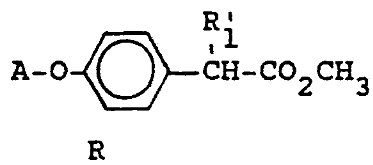

| A | $R/R_1'$ | m.p. (°C) |
|---|---|---|
| 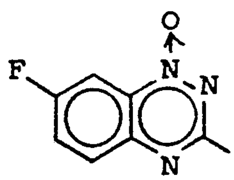 | $H/CH_3$ | 124-127 |

| | | |
|---|---|---|
| 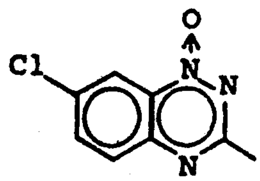 | H/$CH_3$ | 166-169 |
| 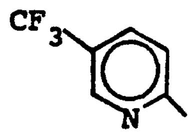 | H/$CH_3$ | oil<br>NMR ($CDCl_3$) δ 1.53 (d, 3H), 3.68 (s, 3H), 3.77 (q, 1H), 7 (d, 1H), 7.11 (d, 2H), 7.37 (d, 2H), 7.9 (dd, 1H), 8.45 (d, 1H). |
| 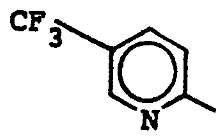 | Cl/$CH_3$ | oil<br>NMR ($CDCl_3$) δ 1.53 (d, 3H), 3.69 (s, 3H), 3.75 (q, 1H), 7.08-7.33 (m, 3H), 7.46 (d, 1H), 7.94 (dd, 1H), 8.4 (d, 1H). |

Table I (continued)

| A | R/R'1 | m.p.(°C) |
|---|---|---|
| 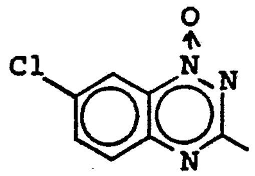 | Cl/$CH_3$ | 151-152 |
| 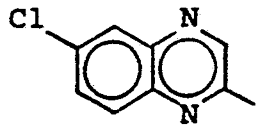 | Cl/$CH_3$ | 84-88 |
| 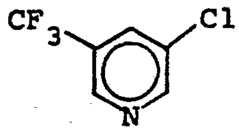 | Cl/$CH_3$ | oil |
| 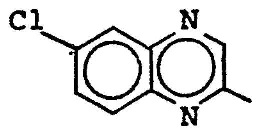 | F/$CH_3$ | 100-101 |
| 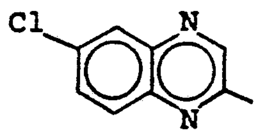 | $OCH_3$/$CH_3$ | 124-126 |
| 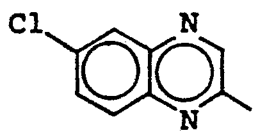 | Cl/$C_2H_5$ | 73-78 |
| 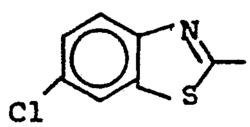 | Cl/$CH_3$ | 123-125 |

Table I (continued)

| A | R/$R_1'$ | m.p. (°C) |
| --- | --- | --- |
| 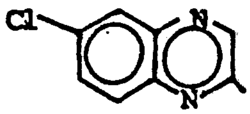 | F/$CH_2$=CH-$CH_2$ | 75-78 |
| 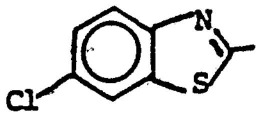 | H/$CH_3$ | 104-106 |
| 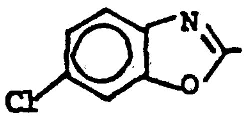 | Cl/$CH_3$ | |
| 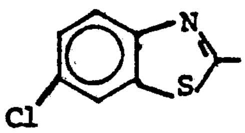 | $OCH_3$/$CH_3$ | |
| 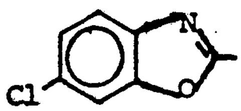 | $OCH_3$/$CH_3$ | |
| 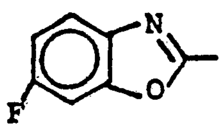 | $OCH_3$/$CH_3$ | |
| 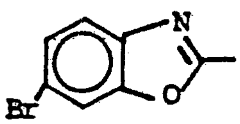 | $OCH_3$/$CH_3$ | |
| 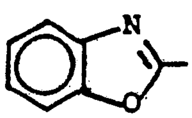 | $OCH_3$/$CH_3$ | |
| 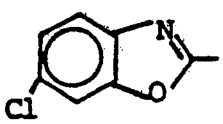 | H/$CH_3$ | |
| 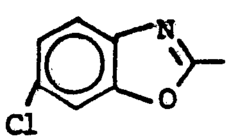 | F/$CH_3$ | |

Table I (continued)

| A | $R/R_1'$ | m.p.(°C) |
|---|---|---|
| 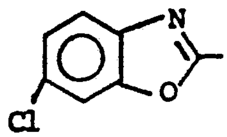 | $Br/CH_3$ | |
| 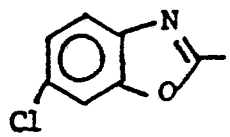 | $CH_3/CH_3$ | |
| 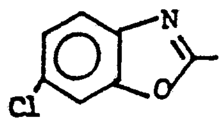 | $OCH_2CH_3/CH_3$ | |
| 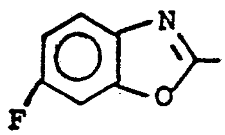 | $F/CH_3$ | |
| 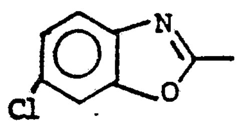 | $Cl/CH_2CH_3$ | |
| 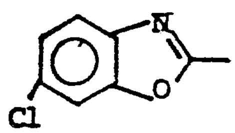 | $Cl/(CH_2)_2CH_3$ | |
| 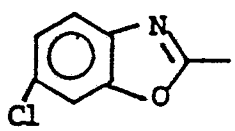 | $Cl/CH_2CH{=}CH_2$ | |
| 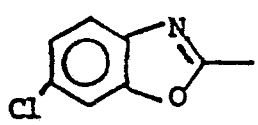 | $CH_2C{\equiv}CH$ | |
| 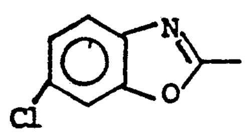 | $CH_3/CH_2CH_3$ | |
| 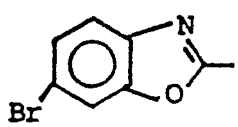 | $Br/CH_2CH_3$ | |
| 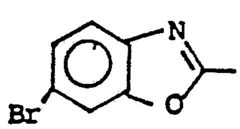 | $CH_3/CH_3$ | |

## Table II

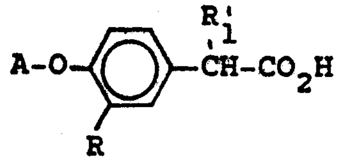

| A | R/$R_1'$ |
|---|---|
| 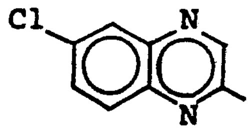 | Cl/$CH_3$ |
| 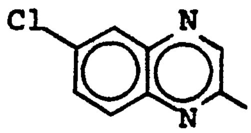 | H/$CH_3$ |
| 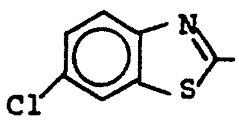 | Cl/$CH_3$ |
| 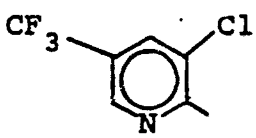 | Cl/$CH_3$ |
| 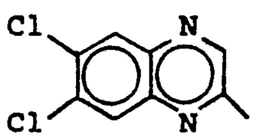 | H/$C_2H_5$ |
| 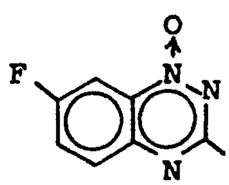 | H/$CH_3$ |
| 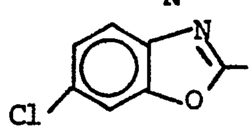 | $OCH_3$/$CH_3$ |

Table III

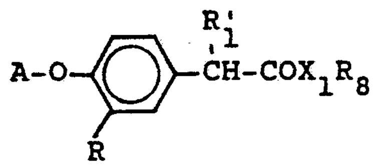

| A | R/R'1 | $X_1R_8$ |
|---|---|---|
| 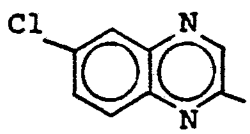 | $Cl/CH_3$ | $-O(CH_2)_3CH_3$ |
| 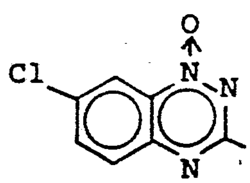 | $H/CH_3$ | 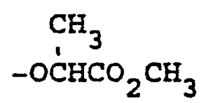 |
| 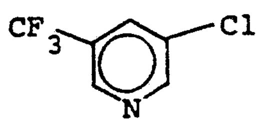 | $H/CH_3$ | 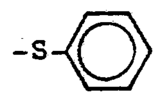 |
| 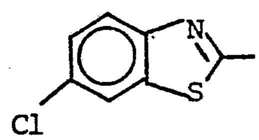 | $CH_3/CH_3$ | $OCH_2CH_2OC_2H_5$ |

**Table III (Continued)**

| | | |
|---|---|---|
| 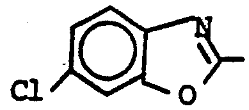 | Cl/$CH_3$ | $OC_2H_5$ |
| 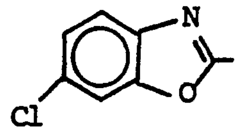 | $OCH_3$/$CH_3$ | $OC_2H_5$ |
| 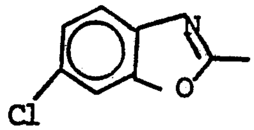 | Cl/$CH_3$ | $OCH_2$– 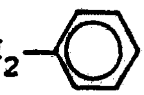 |

Formulations

Useful formulations of the compounds of Formula II can be prepared in conventional ways. They include dusts, granules, pellets, solu- tions, suspensions, emulsions, wettable powders, emul- sifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations may contain active ingredient(s) with at least one of the following: surfactant, solid inert diluent and liquid inert diluent. The formulations, preferably, contain about 0.1% to 99% by weight of active ingredient(s) and at least one of (a) about 0.1% to 20% surfactant(s), (b) about 1% to 99.9% solid inert diluent(s), (c) liquid inert diluent(s) and (d) mixtures of (a), (b) and (c). More specifically, they will contain these ingredients in the following approximate proportions:

**Table IV**

| | Weight Percent* | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a surfactant or a diluent equals 100 weight percent.

Lower or higher levels of active ingredient can, of course, be present depending on the intended use and the physical properties of the compound. Higher rations of surfactant to active ingredient are sometimes

desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through col. 5, line 17 and Examples 1-4;

G. C. Klingman, "Weed Control as a Science", John Wiley and Sons, Inc., New York, pp. 81-96; and

J. D. Fryer and S. A. Evans, "Weed Control Handbook", 5th Ed., Blackwell Scientific Publications, Oxford, 1968, pp. 101-103.

In the following examples, all parts are by weight unless otherwise indicated.

Example 3

Wettable Powder

4-[3-Chloro-5-(trifluoromethyl)-2-pyridinyloxy]-α-methylbenzeneacetic acid, methyl ester 50%
sodium alkylnaphthalenesulfonate 2%
low viscosity methyl cellulose 2%
diatomaceous earth 46%

The ingredients are blended, coarsely hammer-milled and then air-milled to produce particles essentially all below 10 microns in diameter. The product is reblended before packaging.

Example 4

Granule

Wettable Powder of Example 3 5%
attapulgite granules 95%
(U.S.S. 20-40 mesh; 0.84-0.42 mm)

A slurry of wettable powder containing 25% solids is sprayed on the surface of attapulgite granules in a double-cone blender. The granules are dried and packaged.

Example 5

Extruded Pellet

3-Chloro--4-(6-chloro-2-quinoxalinyloxy)-α-methylbenzeneacetic acid, methyl ester 25%
anhydrous sodium sulfate 10%
crude calcium ligninsulfonate 5%
sodium alkylnaphthalenesulfonate 1%
calcium/magnesium bentonite 59%

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 6

Low Strength Granule
3-Chloro-4-(6-chloro-2-quinoxalinyloxy)α-methylbenzeneacetic acid, methyl ester 0.1%
attapulgite granules 99.9%
(U.S.S. 20-40 mesh)

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 7

Granule
4-[3-Chloro-5-(trifluoromethyl)-2-pyridinyloxy]-α-methylbenzeneacetic acid, methyl ester 80%
wetting agent 1%
crude ligninsulfonate salt (containing 5-20% of the natural sugars) 10%
attapulgite clay 9%

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14-100 mesh (1410-149 microns), and packaged for use.

Example 8

Low Strength Granule
3-Chloro-4-(6-chloro-2-quinoxalinyloxy)-α-methylbenzeneacetic acid, methyl ester 1%
N,N-dimethylformamide 9%
attapulgite granules 90%
(U.S.S. 20-40 sieve)

The active ingredient is dissolved in the solvent and the solution is sprayed upon dedusted granules in a double cone blender. After spraying of the solution has been completed, the blender is allowed to run for a short period and then the granules are packaged.

Example 9

Aqueous Suspension
4-[3-Chloro-5-(trifluoromethyl)-2-pyridinyloxy]-α-methylbenzeneacetic acid, methyl ester 40.0%
polyacrylic acid thickener 0.3%
dodecylphenol polyethylene glycol ether 0.5%
disodium phosphate 1.0%
monosodium phosphate 0.5%
polyvinyl alcohol 1.0%
water 56.7%

The ingredients are blended and ground together in a sand mill to produce particles essentially all under 5 microns in size.

Example 10

Solution
3-Chloro--4-(6-chloro-2-quinoxalinyloxy)-α-methylbenzeneacetic acid, sodium salt 5%
Water 95%

The salt is added directly to the water with stirring to produce the solution, which may then be packaged for use.

Example 11

High Strength Concentrate
4-[3-Chloro-5-(trifluoromethyl)-2-pyridinyloxy]-α-methylbenzeneacetic acid, methyl ester 99.0%
silica aerogel 0.5%
synthetic amorphous silica 0.5%

The ingredients are blended and ground in a hammer-mill to produce a material essentially all passing a U.S.S. No. 50 screen (0.3 mm opening). The concentrate may be formulated further if necessary.

Example 12

Wettable Powder
3-Chloro-4-(6-chloro-2-quinoxalinyloxy)-α-methylbenzeacetic acid, methyl ester 90.0%
dioctyl sodium sulfosuccinate 0.1%
synthetic fine silica 9.9%

The ingredients are blended and ground in a hammer-mill to produce particles essentially all below 100 microns. The material is sifted through a U.S.S. No. 50 screen and then packaged.

Example 13

Wettable Powder
3-Chloro--4-(6-chloro-2-quinoxalinyloxy)-α-methylbenzeneacetic acid, methyl ester 40%
sodium ligninsulfonate 20%
montmorillonite clay 40%

The ingredients are thoroughly blended, coarsely hammer-milled and then air-milled to produce particles essentially all below 10 microns in size. the material is reblended and then packaged.

Example 14

Oil Suspension
4-[3-Chloro-5-(trifluoromethyl)-2-pyridinyloxy]-α-methylbenzeneacetic acid, methyl esters%
blend of polyalcohol carboxylic 6%
esters and oil soluble petroleum sulfonates
xylene 59%

The ingredients are combined and ground together in a sand mill to produce particles essentially all below 5 microns. The product can be used directly, extended with oils, or emulsified in water.

Example 15

Dust
3-Chloro-4-(6-chloro-2-quinoxalinyloxy)-α-methylbenzeneacetic acid, methyl ester 10%
attapulgite 10%
Pyrophyllite 80%

The active ingredient is blended with attapulgite and then passed through a hammer-mill to produce particles substantially all below 200 microns. The ground concentrate is then blended with powdered pyrophyllite until homogeneous.

Example 16

Oil Suspension
4-[3-Chloro-5-(trifluoromethyl)-2-pyridinyloxy]-α-methylbenzeneacetic acid, methyl ester 25%
polyoxyethylene sorbitol hexaoleate 5%
highly aliphatic hydrocarbon oil 70%

The ingredients are ground together in a sand mill until the solid particles have been reduced to under about 5 microns. The resulting thick suspension may be applied directly, but preferably after being extended with oils or emulsified in water.

Utility

Test results show that the compounds of the present invention are active herbicides. They are useful for the selective pre- or postemergence weed control in crops, especially in rice, cotton, sugar beets and soybeans.

The rates of application for the compounds of the invention are determined by a number of factors, including the crop species involved, the types of weeds to be controlled, weather and climate, formulations selected, mode of application, amount of foliage present, etc. In general terms, the subject compounds should be applied at levels of around 0.03 to 10 kg/ha, the lower rates being suggested for use on lighter soils and/or those having a low organic matter content or for situations where only short-term persistence is required or for situations where the weed species are easily controlled postemergence.

The compounds of this invention are particularly useful for the control of weeds in rice. They may be used for both paddy and dryland rice. They may be applied postemergence to dryland rice. They may also be applied to paddy rice after transplanting as a spray or granule. The application may be made from 0 to 2 leaf stage of barnyardgrass growth.

Rates of 0.03 to 1 kg/ha will provide weed control. The rate selected for use will depend on the method and timing of application, chemical used, size of weeds, soil type, and other factors. One with ordinary skill in the art can select the rate for any given situation. The compounds are particularly useful for the control of barnyardgrass (Echinochloa crus-galli), a pernicious weed in rice culture, but may also provide complete or

partial control of other weeds, particularly gramineous weeds, in rice.

The compounds of the invention may be used in combination with any other commercial herbicide, examples of which are those of the triazine, triazole, uracil, urea, amide, diphenylether, carbamate, bipyridylium and sulfonylurea types.

In particular, the compounds of this invention may be mixed with other rice herbicides to provide a broader spectrum of weed control, extended control or other benefits. They are especially useful for use in conjunction with sulfonylureas selective in rice such as 2-[[(4,6-dimethoxypyrimidin-2-yl)aminocarbonyl]-aminosulfonylmethyl]benzoic acid, methyl ester, 5-[[(4,6-dimethoxypyrimidin-2-yl)aminocarbonyl]amino sulfonyl]-1-methylpyrazole-4-carboxylic acid, ethyl ester, N-[(4,6-dimethoxy-2-pyrimidinyl)aminocarbonyl]-3-(pentafluoro-1-propenyl)-2-thiophenesulfonamide, 3-(2-chloro-1,2-difluoroethenyl)-N-[(4,6-dimethoxy-2-pyrimidinyl)aminocarbonyl]-2-thiophenesulfonamide, 3-chloro-5-[[(4,6-dimethoxy-2-pyrimidinyl)aminocarbonyl]aminosulfonyl]-1-methyl-1H-pyrazole-4-carboxylic acid, methyl ester and 3-chloro-5-[[(4-methoxy-6-methylpyrimidin-2-yl)aminocarbonyl]aminosulfonyl]-1-methyl-1H-pyrazole-4-carboxylic acid, methyl ester.

The herbicidal properties of the subject compounds were discovered in greenhouse tests. The test procedures and results follow.

Compounds

Compound 1

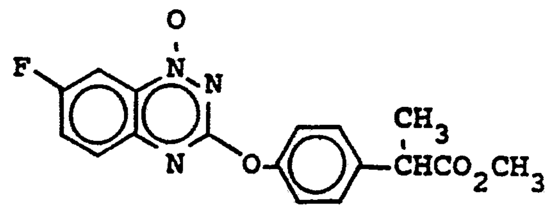

Compound 2

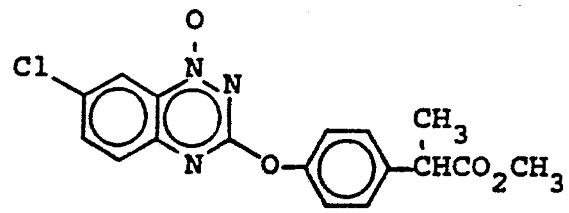

Compound 3

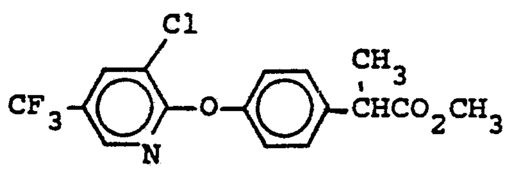

Compound 4

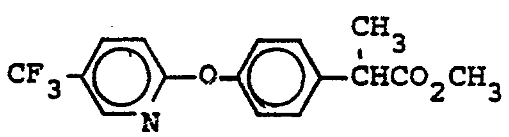

Compound 5

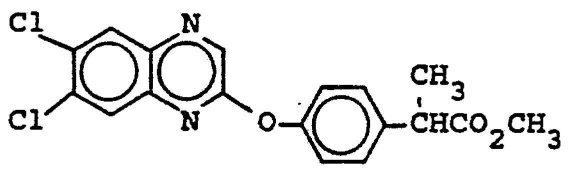

Compound 6

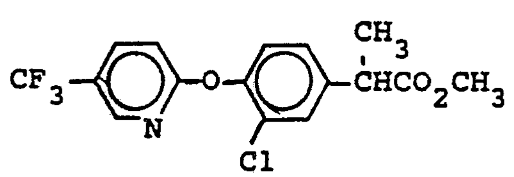

Compounds (continued)

Compound 7

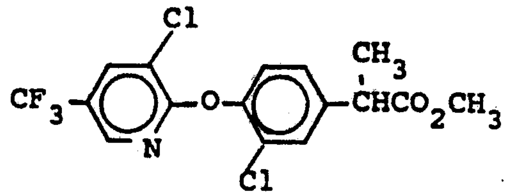

Compound 8

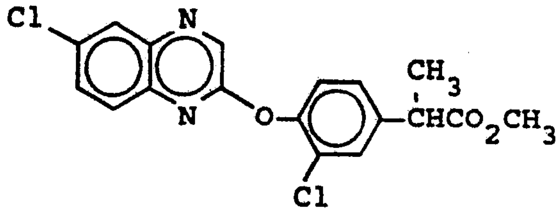

Compound 9

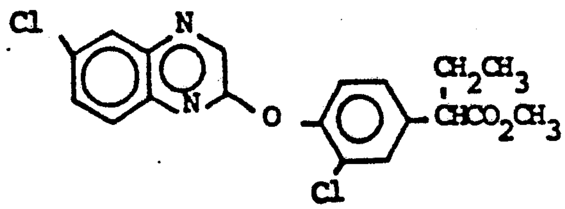

Compound 10

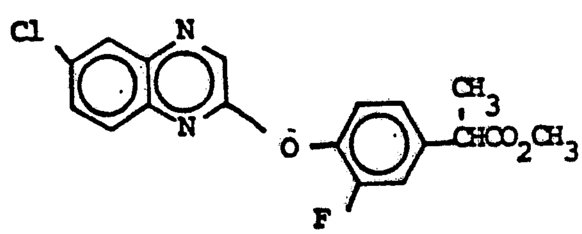

Compounds (continued)

Compound 11

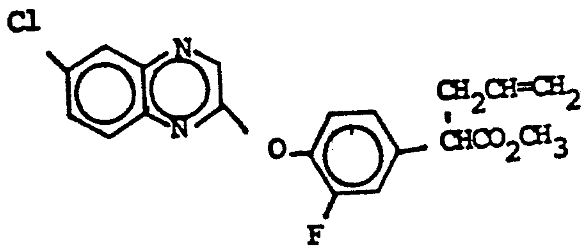

Compound 12

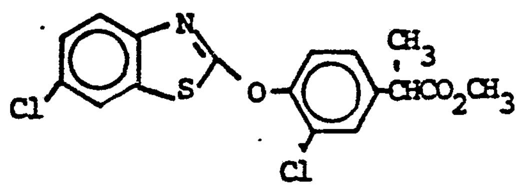

Compound 13

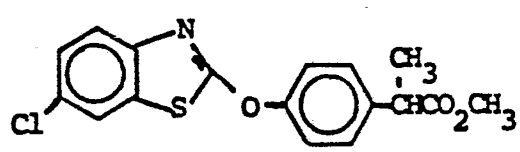

Compounds (continued)

Compound 14

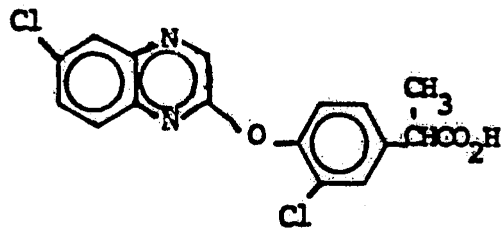

Compound 15

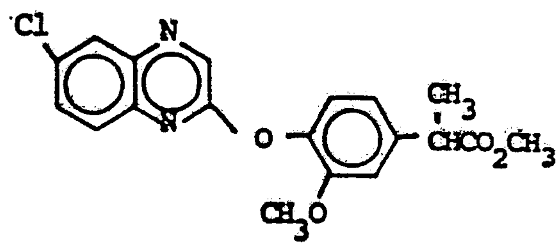

Test A

Seeds of crabgrass (Digitaria sp.), barnyardgrass (Echinochloa crusgalli), wild oats (Avena fatua), velvetleaf (Abutilon theophrasti), morningglory (Ipomoea spp.), cocklebur (Xanthium pensylvanicum), sorghum, corn, soybean, sugarbeet, cotton, rice, wheat and optionally cheatgrass (Bromus secalinus), giant foxtail (Setaria faberii) and sicklepod (Cassia obtusifolia), as well as tubers of purple nutsedge (Cyperus rotundus), were planted and treated preemergence with the test chemicals dissolved in a non-phytotoxic solvent. At the same time, these crop and weed species were treated with a soil/foliage application. At the time of treatment, the plants ranged in height from 2 to 18 cm. Treated plants and controls were maintained in a greenhouse for sixteen days, after which all species were compared to controls and visually rated for response to treatment. The ratings, summarized in Table A, are based on a numerical scale extending from 0 = no injury, to 10 = complete kill. The accompanying descriptive symbols have the following meanings:

B = burn;
C = chlorosis/necrosis;
D = defoliation;
E = emergence inhibition;
G = growth retardation;
H = formative effect;
U = unusual pigmentation;
X = axillary stimulation;
S = albinism; and
6Y = abscised buds or flowers.

Table A (continued)

| | Cmpd. 1 | Cmpd. 2 |
|---|---|---|
| Rate kg/ha | 2 | 2 |
| POSTEMERGENCE | | |
| Morningglory | 0 | 0 |
| Cocklebur | 0 | 0 |
| Velvetleaf | 0 | 0 |
| Nutsedge | 0 | 0 |
| Crabgrass | 2C,6G | - |
| Giant Foxtail | 6G | 0 |
| Barnyardgrass | 7C | 1H |
| Cheatgrass | 0 | 0 |
| Wild Oats | 0 | 0 |
| Wheat | 0 | 2G |
| Corn | 2G | 1H |
| Barley | 0 | 0 |
| Soybean | 3B | 0 |
| Rice | 2C,8G | 2G |
| Sorghum | 1B | 2G |
| Sugar beet | 0 | 0 |
| Cotton | 1B | 0 |
| PREEMERGENCE | | |
| Morningglory | 0 | 0 |
| Cocklebur | 2H | 0 |
| Velvetleaf | 6H | 2G |
| Nutsedge | 0 | 0 |
| Crabgrass | 2C,7G | 9G |
| Giant Foxtail | 0 | 8H |
| Barnyardgrass | 10E | 7H |
| Cheatgrass | 0 | 3G |
| Wild Oats | 2C,5G | 2G |
| Wheat | 5G | 0 |
| Corn | 5G | 2G |
| Barley | 0 | 0 |
| Soybean | 0 | 0 |
| Rice | 8H | 3G |
| Sorghum | 10C | 0 |
| Sugar beet | 0 | 0 |
| Cotton | 0 | 2G |

Table A (continued)

| | Cmpd. 3 | Cmpd. 4 | Cmpd. 5 | Cmpd. 6 |
|---|---|---|---|---|
| Rate kg/ha | 2 | 2 | 2 | 2 |
| POSTEMERGENCE | | | | |
| Morningglory | 2G | 0 | 0 | 0 |
| Cocklebur | 2G | 0 | 0 | 0 |
| Velvetleaf | 5G | 1B | 0 | 0 |
| Nutsedge | 3G | 0 | 0 | 0 |
| Crabgrass | 1B | 3G | 0 | 0 |
| Giant Foxtail | 3B | 5G | 0 | 0 |
| Barnyardgrass | 3B | 0 | 0 | 0 |
| Cheatgrass | 4G | 0 | 0 | 0 |
| Wild Oats | 1B | 0 | 0 | 0 |
| Wheat | 1B | 0 | 0 | 0 |
| Corn | 1B,2H | 2C,3H | 0 | 0 |
| Barley | 0 | 0 | 0 | 0 |
| Soybean | 3B | 3B | 1C,2H | 2G |
| Rice | 2B | 2C,4G | 0 | 0 |
| Sorghum | 0 | 1C,2G | 0 | 2G |
| Sugar beet | 2G | 0 | 0 | 0 |
| Cotton | 2B | 0 | 0 | 2G |
| PREEMERGENCE | | | | |
| Morningglory | 0 | 0 | - | 0 |
| Cocklebur | 2G | 0 | - | 0 |
| Velvetleaf | 2G | 0 | - | 0 |
| Nutsedge | 0 | 0 | - | 0 |
| Crabgrass | 10E | 2C,8G | - | 9H |
| Giant Foxtail | 9H | 10E | - | 9H |
| Barnyardgrass | 9H | 8H | - | 2C,7H |
| Cheatgrass | 7G | 3G | - | 2G |
| Wild Oats | 5G | 4G | - | 0 |
| Wheat | 2G | 3G | - | 0 |
| Corn | 2G | 3G | - | 0 |
| Barley | 2G | 5G | - | 0 |
| Soybean | 5G | 5G | - | 0 |
| Rice | 2G | 4G | - | 0 |
| Sorghum | 8H | 3C,9H | - | 5G |
| Sugar beet | 8G | 4G | - | 3G |
| Cotton | 0 | 2G | - | 0 |

Table A (continued)

| | Cmpd. 7 | Cmpd. 8 |
|---|---|---|
| Rate kg/ha | 2 | 2 |
| POSTEMERGENCE | | |
| Morningglory | 0 | 1B |
| Cocklebur | 0 | 1B |
| Velvetleaf | 1B | 0 |
| Nutsedge | 0 | 0 |
| Crabgrass | 1B | 9C |
| Giant Foxtail | 1H | 9C |
| Barnyardgrass | 8H | 10C |
| Cheatgrass | 0 | 3C |
| Wild Oats | 0 | 5G |
| Wheat | 0 | 2C,7G |
| Corn | 0 | 4C,9H |
| Barley | 0 | 9C |
| Soybean | 0 | 0 |
| Rice | 0 | 2G |
| Sorghum | 1B | 9C |
| Sugar beet | 0 | 0 |
| Cotton | 1B | 1B |
| PREEMERGENCE | | |
| Morningglory | 0 | 0 |
| Cocklebur | 0 | 4G |
| Velvetleaf | 0 | 0 |
| Nutsedge | 0 | 0 |
| Crabgrass | 9H | 9H |
| Giant Foxtail | 10E | 10H |
| Barnyardgrass | 5C,9H | 10H |
| Cheatgrass | 0 | 8G |
| Wild Oats | 0 | 0 |
| Wheat | 2G | 0 |
| Corn | 0 | 9C |
| Barley | 0 | 8G |
| Soybean | 0 | 0 |
| Rice | 2G | 0 |
| Sorghum | 0 | 9H |
| Sugar beet | 2G | 0 |
| Cotton | 0 | 0 |

Table A (continued)

| | Cmpd. 9 | Cmpd. 10 | Cmpd. 11 | Cmpd. 12 |
|---|---|---|---|---|
| Rate kg/ha | 2 | 2 | 2 | 2 |
| POSTEMERGENCE | | | | |
| Morningglory | 0 | 0 | 0 | 1B |
| Cocklebur | 0 | 0 | 0 | 1B |
| Velvetleaf | 0 | 0 | 0 | 0 |
| Nutsedge | 0 | 0 | 0 | 0 |
| Crabgrass | 0 | 9C | 2G | 6C |
| Giant Foxtail | 5G | 9C | 3G | 7C |
| Barnyardgrass | 7H | 10C | 5C,9H | 10C |
| Cheatgrass | 0 | 2G | 0 | 0 |
| Wild Oats | 0 | 3C,7G | 0 | 0 |
| Wheat | 0 | 3C,7G | 0 | 0 |
| Corn | 0 | 9C | 0 | 0 |
| Barley | 0 | 9C | 0 | 2C |
| Soybean | 0 | 3G | 0 | 1B |
| Rice | 2G | 0 | 0 | 0 |
| Sorghum | 0 | 7H | 0 | 0 |
| Sugar Beet | 0 | 2G | 0 | 0 |
| Cotton | 0 | 0 | 0 | 2C |
| PREEMERGENCE | | | | |
| Morningglory | 0 | 0 | 0 | 0 |
| Cocklebur | 0 | 0 | 0 | 0 |
| Velvetleaf | 0 | 0 | 0 | 0 |
| Nutsedge | 0 | 0 | 0 | 9G |
| Crabgrass | 8G | 10H | 0 | 3G |
| Giant Foxtail | 9H | 10H | 7H | 7C |
| Barnyardgrass | 9H | 10H | 9H | 9C |
| Cheatgrass | 0 | 6G | 0 | 0 |
| Wild Oats | 0 | 0 | 0 | 0 |
| Wheat | 0 | 4G | 0 | 0 |
| Corn | 0 | 3C,8H | 0 | 0 |
| Barley | 0 | 7G | 0 | 0 |
| Soybean | 0 | 2C,2H | 0 | 0 |
| Rice | 2G | 0 | 0 | 0 |
| Sorghum | 0 | 3C,8H | 0 | 0 |
| Sugar beet | 3G | 0 | 0 | 0 |
| Cotton | - | 0 | 0 | 0 |

Table A (continued)

| | Cmpd. 13 | Cmpd. 14 | Cmpd. 15 |
|---|---|---|---|
| Rate kg/ha | 2 | 2 | 2 |
| POSTEMERGENCE | | | |
| Morningglory | 1B | 0 | 0 |
| Cocklebur | 0 | 0 | 0 |
| Velvetleaf | 0 | 0 | 0 |
| Nutsedge | 0 | 0 | 0 |
| Crabgrass | 8C | 0 | 9C |
| Giant Foxtail | 9C | 2C,3G | 7C |
| Barnyardgrass | 10C | 3C,8H | 10C |
| Cheatgrass | 6C | 0 | 6C |
| Wild Oats | 2C | 0 | 8C |
| Wheat | 0 | 0 | 6C |
| Corn | 1C | 2C,4H | 9C |
| Barley | 4C | 0 | 5C,9G |
| Soybean | 1B | 0 | 0 |
| Rice | 0 | 0 | 1S,3G |
| Sorghum | 0 | 0 | 1S,4G |
| Sugar Beet | 0 | 0 | 0 |
| Cotton | 1C | 2G | 3G |
| PREEMERGENCE | | | |
| Morningglory | 0 | 5H | 0 |
| Cocklebur | 0 | 0 | 0 |
| Velvetleaf | 0 | 0 | 2G |
| Nutsedge | 10E | 5G | 0 |
| Crabgrass | 8G | 9G | 9H |
| Giant Foxtail | 5C | 10H | 10H |
| Barnyardgrass | 9C | 10E | 10H |
| Cheatgrass | 0 | 6G | 0 |
| Wild Oats | 0 | 3H | 0 |
| Wheat | 0 | 3H | 0 |
| Corn | 3H | 4C,9H | 0 |
| Barley | 0 | 3C,8G | 0 |
| Soybean | 0 | 0 | 0 |
| Rice | 0 | 4G | 0 |
| Sorghum | 0 | 2G | 1H |
| Sugar beet | 0 | 0 | 0 |
| Cotton | 0 | 0 | 0 |

Test B

Sixteen cm diameter Wagenr pots, equipped with a stoppered drain opening near the bottom of the side wall, were partially filled with Woodstown sandy loam. About 1500 mls of water were added to each pot to bring the water level to a point 3 cm above the soil surface. Japonica and Indica rice seedlings were transplanted as described in Test E. Also, a number of barnyardgrass (Echinochloa crus-galli) seeds were added to each pot. At the same time, seedlings or tubers of the following species were transplanted into the muddy soil: water plantain (Alisma trivale), Scirpus (Scirpus mucranatus), and Cyperus (Cyperus difformis). The weed species selected for this test are of economic importance in major rice-growing areas. The chemical treatments were applied directly to the paddy water after being formulated in a non-phytotoxic solvent within hours after transplanting of two additional species: water chestnut (Eleocharis spp.) and arrowhead (Sagittaria latifolia). Shortly after treatment, the drain hole was opened to drop the water level by two cm. Water was then added to restore the water level to its original height. The following day the draining and refilling process was repeated. The pots were then maintained in the greenhouse. Rates of application and plant response ratings made 21 days after treatment are summarized in Table B. The ratings are percentages where 0 = no injury and 100 = complete kill.

In the subsequent tables, LS is used as an abbreciation for leaf stage.

Table B

Compound 5

| Rate (g/ha) | J | I | BYG | WC | A | SC | CY | WP |
|---|---|---|---|---|---|---|---|---|
| | % Injury or Control (average of 2 reports) | | | | | | | |
| 250 | 0 | 0 | 95 | - | 70 | 25 | 0 | 0 |
| 1000 | 0 | 0 | 100 | - | 0 | 0 | 0 | 0 |

Compound 8

| Rate (g/ha) | J | I | BYG | WC | A | SC | CY | WP |
|---|---|---|---|---|---|---|---|---|
| | % Injury or Control (average of 2 reports) | | | | | | | |
| 500 | 0 | 7 | 100 | 0 | 0 | 100 | 0 | 0 |
| 1000 | 5 | 12 | 100 | 0 | 0 | 0 | 0 | 0 |
| 2000 | 5 | 0 | 100 | 0 | 0 | 0 | 0 | 0 |

J = Japonica Rice
I = Indica Rice
BYG = Barnyardgrass
WC = Water chestnut
A = Arrowhead
SC = *Scirpus*
CY = *Cyperus*
WP = Water Plantain

Table C

Compound 5

% Injury (average of 2 reports)

| Rate (g/ha) | 0.25 | 1 | 4 | 16 | 30 | 63 | 125 | 250 | 500 | 1000 | 2000 |
|---|---|---|---|---|---|---|---|---|---|---|---|
| Japonica | 30 | 20 | 0 | 35 | 0 | 30 | 0 | 30 | 0 | 35 | 15 |
| Indica | 0 | 45 | 0 | 0 | 0 | 0 | 35 | 45 | 35 | 40 | 35 |

Compound 8

% Injury (average of 2 reports)

| Rate (g/ha) | 30 | 125 | 500 | 1000 | 2000 |
|---|---|---|---|---|---|
| Japonica | 0 | 0 | 0 | 0 | 10 |
| Indica | 0 | 0 | 0 | 0 | 0 |

Test D

Sixteen cm diameter Airlite plastic pots were partially filled with Tama silt loam soil and the soil saturated with water. Japonica and Indica rice seedlings at the 2.0 to 2.5 leaf stage were transplanted into 1/3 of the pots. Into another third of the pots were transplanted seedling or sprouted tubers of water plantain (Alisma trivale), Scripus (Scirpus paludosus), Cyperus (Cyperus esculentus), and arrowhead (Saggittaria spp.). The remaining pots were planted with barnyardgrass (Echinochloa crusgalli) seeds and sprouted tubers of water chestnut (Eleocharis spp.). These weeds all represent major rice weeds or genera of weeds important in rice. Three to four days after planting, the water level was raised to 3 cm (about 1200 ml/pot) and maintained at this level throughout the test. Chemical treatments were applied directly to the paddy water, within 24 hours of raising the water, after being formulated in a non-phytotoxic solvent. The pots were maintained in the greenhouse. Plant response ratings were made 21 days after treatment.

Response ratings are based on a scale of 0 to 100 where 0 = no effect, and 100 = complete control. A blank means no test.

Rates of application and plant response ratings are summarized in Table L.

While Compound 28 exhibited little activity at the application rates tested in this trial, it is evident from the results of Test A that it is herbicidally active at higher application rates.

## TABLE D

CMPD 12

| RATE=G/HA | 0064 | 0125 | 0250 | 0500 | 1000 |
|---|---|---|---|---|---|
| BARNYARD GRASS | 85 | 85 | 70 | 80 | 100 |
| WATER CHESTNUT | 0 | 0 | 0 | 0 | 0 |
| ARROWHEAD | 0 | 0 | 0 | 0 | 0 |
| SCIRPUS (SEDGE) | 0 | 0 | 0 | 0 | 0 |
| CYPERUS (SEDGE) | 0 | 0 | 0 | 0 | 0 |
| WATER PLANTAIN | 0 | 0 | 0 | 0 | 0 |
| RICE JAPONICA | 0 | 0 | 0 | 0 | 0 |
| RICE INDICA | 0 | 0 | 0 | 0 | 0 |

CMPD 13

| RATE=G/HA | 0064 | 0125 | 0250 | 0500 | 1000 |
|---|---|---|---|---|---|
| BARNYARD GRASS | 95 | 90 | 85 | 100 | 100 |
| WATER CHESTNUT | 0 | 0 | 0 | 0 | 0 |
| ARROWHEAD | 0 | 0 | 0 | 0 | 0 |
| SCIRPUS (SEDGE) | 0 | 0 | 0 | 0 | 0 |
| CYPERUS (SEDGE) | 0 | 0 | 0 | 0 | 0 |
| WATER PLANTAIN | 0 | 0 | 0 | 0 | 0 |
| RICE JAPONICA | 0 | 0 | 0 | 0 | 0 |
| RICE INDICA | 0 | 0 | 0 | 0 | 0 |

CMPD 14

| RATE=G/HA | 0064 | 0125 | 0250 | 0500 | 1000 |
|---|---|---|---|---|---|
| BARNYARD GRASS | 0 | 0 | 0 | 0 | 0 |
| WATER CHESTNUT | 0 | 0 | 0 | 0 | 0 |
| ARROWHEAD | 0 | 0 | 0 | 0 | 0 |
| SCIRPUS (SEDGE) | 0 | 0 | 0 | 0 | 0 |
| CYPERUS (SEDGE) | 0 | 0 | 0 | 0 | 0 |
| WATER PLANTAIN | 0 | 0 | 0 | 0 | 0 |
| RICE JAPONICA | 0 | 0 | 0 | 0 | 0 |
| RICE INDICA | 0 | 0 | 0 | 0 | 0 |

CMPD 15

| RATE=G/HA | 0064 | 0125 | 0250 | 0500 | 1000 |
|---|---|---|---|---|---|
| BARNYARD GRASS | 0 | 60 | 100 | 100 | 100 |
| WATER CHESTNUT | 0 | 0 | 0 | 0 | 0 |
| ARROWHEAD | 0 | 0 | 0 | 0 | 0 |
| SCIRPUS (SEDGE) | 0 | 0 | 0 | 0 | 0 |
| CYPERUS (SEDGE) | 0 | 0 | 0 | 0 | 0 |
| WATER PLANTAIN | 0 | 0 | 0 | 0 | 0 |
| RICE JAPONICA | 0 | 0 | 0 | 0 | 10 |
| RICE INDICA | 0 | 0 | 0 | 0 | 20 |

Test E

Postemergence

Two round pans (25 cm diameter by 12.5 cm deep wre filled with Sassafras sandy loam soil. One pan was planted with blackgrass (Aloperurus myosuroides), sugarbeets, nutsedge (Cyperus rotundus) tubers, rape (Brassica napus), crabgrass (Digitaria sanguinalis), sicklepod (cassia obtusifolia), teaweed (Sida spinosa), jimsonweed (Datura stramonium), velvetleaf (Abtilon theophrast), and giant foxtail (Setaria faberii). The other pan was planted with wheat, cotton, rice, corn, soybean, wild oats (Avena fatua), cocklebur (Xantium ensylvanicum), morningglofy (Ipomoea hederacea), johnsongrass (Sorghum halepense) and barnyardgrass (Echinochloa crus-galli). The plants were grown for approximately fourteen days, then sprayed postemergence with the chemicals dissolved in a non-phytotoxic solvent.

Preemergence

Two round pans (25 cm diameter by 12.5 cm deep) were filled with Sassafras sandy loam soil. One pan was planted with blackgrass, sugarbeets, nutsedge, rape, crabgrass, sicklepod, teaweed, jimsonweed, velvetleaf, and giant foxtail. The other pan was planted with wheat, cotton, rice, cron, soybeans, wild oats, cocklebur, morningglory, johnsongrass, and barnyardgrass. The two pans were sprayed preemergence with the chemicals dissolved in a non-phytotoxic solvent.

Treated plants and controls were maintained in the greenhouse for 28 days, then all treated plants were compared to controls and visually rated for plant response.

Response ratings are based on a scale of 0 to 100 where 0 = no effect, and 100 = complete control. A dash (-) response means no test.

response ratings are contained in Table M.

## Table E

## Compound 8

| | POSTEMERGENCE | | PREEMERGENCE | | |
|---|---|---|---|---|---|
| Rate g/ha | 62 | 16 | 250 | 62 | 16 |
| Corn | 0 | 0 | 0 | 0 | 0 |
| Wheat | 0 | 0 | 0 | 0 | 0 |
| Barley | 0 | 0 | 0 | 0 | 0 |
| Rice | 0 | 0 | 0 | 0 | 0 |
| Soybean | 0 | 0 | 0 | 0 | 0 |
| Cotton | 0 | 0 | - | - | - |
| Sugarbeet | 0 | 0 | 0 | 0 | 0 |
| Rape | 0 | 0 | 60 | 30 | 0 |
| Crabgrass | 50 | 30 | 60 | 30 | 0 |
| Johnsongrass | 50 | 30 | 0 | 0 | 0 |
| Blackgrass | 0 | 0 | 50 | 30 | 0 |
| Barnyardgrass | 30 | 0 | 0 | 0 | 0 |
| Nutsedge | 50 | 30 | 50 | 30 | 0 |
| Giant Foxtail | 30 | 0 | 0 | 0 | 0 |
| Green Foxtail | 30 | 0 | 0 | 0 | 0 |
| Cheatgrass | 0 | 0 | 0 | 0 | 0 |
| Wild Oats | 0 | 0 | 0 | 0 | 0 |
| Wild Buckwheat | 0 | 0 | 50 | 30 | 0 |
| Viola | 0 | 0 | 0 | 0 | 0 |
| Lambsquarter | 0 | 0 | 0 | 0 | 0 |
| Cocklebur | 0 | 0 | 0 | 0 | 0 |
| Morningglory | 50 | 30 | 0 | 0 | 0 |
| Teaweed | 0 | 0 | 70 | 50 | 30 |
| Cassia | 0 | 0 | 30 | 0 | 0 |
| Jimsonweed | 30 | 0 | 30 | 0 | 0 |
| Velvetleaf | 0 | 0 | 0 | 0 | 0 |

Test F

Postemergence

Three round pans (25 cm diameter by 12.5 cm deep) were filled with Sassafras sandy laom soil. One pan was planted with nutsedge (Cyperus rotundus) tubers, crabgrass (Digitaria sanguinalis), sicklepod (Cassia obtusifolia), jimisonweed (Datura stramonium), velvetleaf (Abutilon theophrasti), lambsquarters (Chenopodium album), rice (Oryza sativa), and teaweed (Sida spinosa). The second pot was planted with green foxtail (Setaria viridis), cocklebur (Xanthium pensylvanicum), morningglory (Ipomoea hederacea), cotton (Gossypium hirsutum), johnsongrass (Sorghum halepense), barnyardgrass (Echinochloa crus-galli), corn (Zea mays), soybean (Glycine max), and giant foxtail (Setaria faberi). The third pot was planted with wheat (Triticum aestivum), barley (Hordeum vulgare), wild buckwheat (Polygonum convolvulus L.), cheatgrass (Bromus secalinus L.), sugarbeet (Beta vulgaris), wild oat (Avena fatua L.), viola (Viola arvenis), blackgrass (Alopecurus myosuroides), and rape (Brassica napus). The plants were grown for approximately fourteen days, then sprayed postemergence with the chemicals dissolved in a nonphytotoxic solvent.

Preemergence

Three round pans (25 cm diameter by 12.5 cm deep) were filled with Sassafras sandy loam soil. One pan was planted with nutsedge tubers, crabgrass, sicklepod, jimsonweed, velvetleaf, lambsquarters, rice, and teaweed. The second pot was plant with green foxtail, cocklebur, morningglory, cotton, johnsongrass, barnyardgrass, corn, soybean, and giant foxtail. The third pot was planted with wheat, barley, wild buckwheat, cheatgrass, sugarbeet, wild oat, viola, blackgrass, and rape. The three pans were sprayed preemergence with the chemicals dissolved in a non-phytotoxic solvent.

Treated plants and controls were maintained in the greenhouse for approximately 24 days, then all rated plants were compared to controls and visually rated for plant response.

Response ratings are based on a scale of 0 to 100 where 0 = no effect, and 100 = complete control. A blank means no test.

Response ratings are contained in Table N.

TABLE F

CMPD 12

| RATE=G/HA | 0250 | 0500 | 1000 | 2000 |
|---|---|---|---|---|
| POST | | | | |
| GIANT FOXTAIL | 0 | 20 | 50 | |
| VELVETLEAF | 0 | 0 | 0 | 0 |
| SUGAR BEETS | 0 | 0 | 40 | 50 |
| CRABGRASS | 0 | 0 | 0 | 0 |
| TEAWEED | 0 | 0 | 0 | 30 |
| JIMSONWEED | 0 | 0 | 0 | 0 |
| RICE | 0 | 0 | 0 | 0 |
| COCKLEBUR | 0 | 0 | 0 | 30 |
| COTTON | 0 | 0 | 20 | 30 |
| SOYBEAN | 0 | 0 | 0 | 0 |
| BARNYARD GRASS | 90 | 100 | 100 | 100 |
| WILD OATS | 0 | 0 | 20 | 30 |
| MORNINGGLORY | 0 | 0 | 0 | 20 |
| WHEAT | 0 | 0 | 0 | 20 |
| CASSIA | 0 | 0 | 20 | 40 |
| JOHNSONGRASS | 0 | 0 | 0 | 0 |
| NUTSEDGE | 0 | 0 | 0 | 0 |
| CORN | 0 | 0 | 0 | 0 |
| WILD BUCKWHEAT | 0 | 0 | 0 | 0 |
| BLACK GRASS | 0 | 0 | 0 | 0 |
| RAPESEED | 0 | 0 | 0 | 0 |
| BARLEY | 0 | 0 | 0 | 30 |
| GREEN FOXTAIL | 0 | 30 | 90 | |
| CHEAT GRASS | 0 | 0 | 0 | 0 |
| VIOLA | 0 | 0 | 0 | 0 |
| LAMBSQUARTER | 0 | 0 | 0 | 0 |
| PRE | | | | |
| GIANT FOXTAIL | 0 | 0 | 0 | |
| VELVETLEAF | 0 | 0 | 0 | 30 |
| SUGAR BEETS | 0 | 0 | 0 | 0 |
| CRABGRASS | 0 | 0 | 30 | 30 |
| TEAWEED | 0 | 0 | 0 | 50 |
| JIMSONWEED | 0 | 0 | 0 | 30 |
| RICE | 0 | 0 | 0 | 0 |
| COCKLEBUR | 0 | 0 | 0 | 0 |
| COTTON | 0 | 0 | 0 | 0 |
| SOYBEAN | 0 | 0 | 0 | 0 |
| BARNYARD GRASS | 100 | 100 | 100 | 100 |
| WILD OATS | 0 | 0 | 0 | 0 |
| MORNINGGLORY | 0 | 0 | 0 | 0 |
| WHEAT | 0 | 0 | 0 | 0 |
| CASSIA | 0 | 0 | 0 | 0 |
| JOHNSONGRASS | 0 | 0 | 0 | 0 |
| NUTSEDGE | 0 | 0 | 0 | 0 |
| CORN | 0 | 0 | 0 | 20 |
| WILD BUCKWHEAT | 0 | 0 | 0 | 0 |
| BLACK GRASS | 0 | 0 | 0 | 40 |
| RAPESEED | 0 | 0 | 0 | 0 |
| BARLEY | 0 | 0 | 0 | 0 |
| GREEN FOXTAIL | 0 | 0 | 30 | 40 |
| CHEAT GRASS | 0 | 0 | 0 | 0 |
| VIOLA | 0 | 0 | 0 | 0 |
| LAMBSQUARTER | 0 | 0 | 0 | 0 |

## TABLE F (continued)

### CMPD 13

| RATE=G/HA | 0250 | 0500 | 1000 | 2000 |
|---|---|---|---|---|
| POST | | | | |
| GIANT FOXTAIL | 0 | 30 | 40 | 80 |
| VELVETLEAF | 0 | 0 | 0 | 0 |
| SUGAR BEETS | 0 | 0 | 0 | 30 |
| CRABGRASS | 0 | 0 | 0 | 30 |
| TEAWEED | 0 | 0 | 0 | 0 |
| JIMSONWEED | 0 | 0 | 0 | 0 |
| RICE | 0 | | 0 | 20 |
| COCKLEBUR | 0 | 0 | 0 | 0 |
| COTTON | 0 | 0 | 0 | 0 |
| SOYBEAN | 0 | 0 | 0 | 0 |
| BARNYARD GRASS | 100 | 100 | 100 | 100 |
| WILD OATS | 0 | 0 | 0 | 30 |
| MORNINGGLORY | 0 | 0 | 0 | 20 |
| WHEAT | 0 | 0 | 0 | 0 |
| CASSIA | 0 | 0 | 0 | 0 |
| JOHNSONGRASS | 0 | 0 | 0 | 0 |
| NUTSEDGE | 0 | 0 | 0 | 0 |
| CORN | 0 | 0 | 0 | 20 |
| WILD BUCKWHEAT | 0 | 0 | 40 | 50 |
| BLACK GRASS | 40 | 50 | 60 | 70 |
| RAPESEED | | 0 | 20 | 20 |
| BARLEY | 20 | 30 | 30 | 40 |
| GREEN FOXTAIL | 20 | 60 | 60 | 90 |
| CHEAT GRASS | 0 | 0 | 0 | 0 |
| VIOLA | 0 | 0 | 30 | 40 |
| LAMBSQUARTER | 0 | 0 | 20 | 50 |
| PRE | | | | |
| GIANT FOXTAIL | 0 | 20 | 70 | 70 |
| VELVETLEAF | 0 | 0 | 0 | 0 |
| SUGAR BEETS | 0 | 0 | 0 | 0 |
| CRABGRASS | 30 | 30 | 50 | 90 |
| TEAWEED | 0 | 0 | 70 | 90 |
| JIMSONWEED | 0 | 0 | 30 | 90 |
| RICE | 0 | 0 | 0 | 0 |
| COCKLEBUR | 0 | 0 | 30 | |
| COTTON | 0 | 0 | 0 | 0 |
| SOYBEAN | 0 | 0 | 0 | 0 |
| BARNYARD GRASS | 90 | 100 | | 100 |
| WILD OATS | 0 | 0 | 0 | 20 |
| MORNINGGLORY | 0 | 0 | 0 | 0 |
| WHEAT | 0 | 0 | 0 | 0 |
| CASSIA | 0 | 0 | 0 | 0 |
| JOHNSONGRASS | 0 | 30 | 40 | 40 |
| NUTSEDGE | 0 | 0 | 0 | 0 |
| CORN | 0 | 0 | 0 | 20 |
| WILD BUCKWHEAT | 0 | 0 | 0 | 0 |
| BLACK GRASS | 20 | 50 | 80 | 90 |
| RAPESEED | 0 | 0 | 0 | 0 |
| BARLEY | 0 | 0 | 0 | 0 |
| GREEN FOXTAIL | 0 | 0 | 30 | 100 |
| CHEAT GRASS | 0 | 0 | 0 | 0 |
| VIOLA | 0 | 0 | 0 | 0 |
| LAMBSQUARTER | | 0 | 0 | 0 |

## TABLE F (continued)

### CMPD 14

| RATE=G/HA | 0250 | 0500 | 1000 |
|---|---|---|---|
| PRE | | | |
| GIANT FOXTAIL | 0 | 30 | 100 |
| VELVETLEAF | 0 | 0 | 0 |
| SUGAR BEETS | 0 | 0 | 0 |
| CRABGRASS | 20 | 40 | 100 |
| TEAWEED | 0 | 0 | 0 |
| JIMSONWEED | 0 | 0 | 0 |
| RICE | 0 | 0 | 0 |
| COCKLEBUR | 0 | | 0 |
| COTTON | 0 | 0 | 0 |
| SOYBEAN | 0 | 0 | 0 |
| BARNYARD GRASS | 100 | 100 | 100 |
| WILD OATS | 0 | 0 | 0 |
| MORNINGGLORY | 0 | 0 | 0 |
| WHEAT | 0 | 0 | 0 |
| CASSIA | 0 | 0 | 0 |
| JOHNSONGRASS | 0 | 0 | 30 |
| NUTSEDGE | 0 | 30 | 30 |
| CORN | 0 | 0 | 0 |
| WILD BUCKWHEAT | 0 | 0 | 0 |
| BLACK GRASS | 0 | 30 | 30 |
| RAPESEED | 0 | 0 | 0 |
| BARLEY | 0 | 0 | 0 |
| GREEN FOXTAIL | 0 | 30 | 90 |
| CHEAT GRASS | 0 | 20 | 30 |
| VIOLA | 0 | 0 | 0 |
| LAMBSQUARTER | 0 | 0 | 0 |

TABLE F (continued)

CMPD 15

| RATE=G/HA | 0062 | 0250 | 0500 | 1000 |
|---|---|---|---|---|
| POST | | | | |
| GIANT FOXTAIL | 0 | 0 | 30 | 30 |
| VELVETLEAF | | 0 | 40 | 80 |
| SUGAR BEETS | 0 | 0 | 0 | 0 |
| CRABGRASS | 30 | 40 | 50 | 60 |
| TEAWEED | 0 | 30 | 30 | 60 |
| JIMSONWEED | 0 | 0 | 0 | 0 |
| RICE | | 20 | 20 | 60 |
| COCKLEBUR | 0 | 0 | 0 | 0 |
| COTTON | 0 | 0 | 10 | 20 |
| SOYBEAN | 0 | 0 | 0 | 0 |
| BARNYARD GRASS | 0 | 90 | 90 | 100 |
| WILD OATS | 0 | 20 | 20 | 30 |
| MORNINGGLORY | 0 | 0 | 40 | 40 |
| WHEAT | 0 | 0 | 0 | 0 |
| CASSIA | 0 | 0 | 30 | 30 |
| JOHNSONGRASS | 30 | 40 | 50 | 60 |
| NUTSEDGE | 0 | 0 | 30 | 40 |
| CORN | 0 | 0 | 0 | 20 |
| WILD BUCKWHEAT | 0 | 0 | 0 | 0 |
| BLACK GRASS | 0 | 0 | 20 | 30 |
| RAPESEED | 0 | 0 | 0 | 0 |
| BARLEY | 10 | | 20 | 20 |
| GREEN FOXTAIL | 0 | 0 | 20 | 40 |
| CHEAT GRASS | 0 | 0 | 0 | 0 |
| VIOLA | 0 | 0 | 0 | 0 |
| LAMBSQUARTER | 0 | 0 | 30 | 30 |

TABLE F (continued)

CMPD 15

| RATE=G/HA | 0062 | 0250 | 0500 | 1000 |
|---|---|---|---|---|
| PRE | | | | |
| VELVETLEAF | 30 | 30 | | 40 |
| SUGAR BEETS | | 0 | 0 | 0 |
| CRABGRASS | | 60 | | 80 |
| TEAWEED | 0 | 0 | 60 | 70 |
| JIMSONWEED | 20 | 30 | 60 | |
| RICE | 0 | 0 | 0 | 0 |
| COCKLEBUR | | 0 | 0 | 20 |
| COTTON | 0 | 0 | 0 | 0 |
| SOYBEAN | 0 | 0 | 0 | 0 |
| BARNYARD GRASS | 0 | 40 | 100 | 100 |
| WILD OATS | 0 | 0 | 0 | 30 |
| MORNINGGLORY | | | 0 | 0 |
| WHEAT | | 0 | 20 | 20 |
| CASSIA | | 0 | 0 | 30 |
| JOHNSONGRASS | 0 | | 0 | 40 |
| NUTSEDGE | 0 | 0 | 20 | 30 |
| CORN | 0 | 0 | 0 | 0 |
| WILD BUCKWHEAT | 0 | 0 | 0 | 0 |
| BLACK GRASS | 0 | | 0 | 30 |
| RAPESEED | | 0 | 0 | 0 |
| BARLEY | | 0 | 10 | 10 |
| GREEN FOXTAIL | 20 | 100 | | 100 |
| CHEAT GRASS | 0 | 0 | 0 | 0 |
| VIOLA | 0 | 0 | 0 | 20 |
| LAMBSQUARTER | | | | 20 |

**Claims**

1. Compounds of Formual II

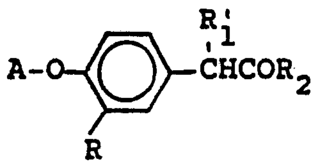

II

where

A is 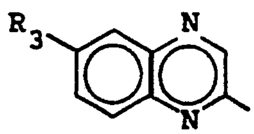 , 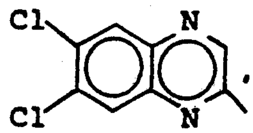 , ,

A-1 A-2

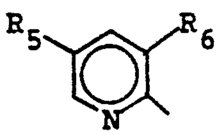 , 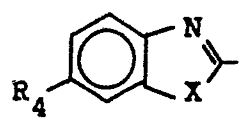 or 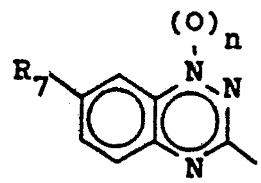 ;

A-4 A-5 A-6

EMI PA=54 FR=1 HE=145 WI=105 TI=CHE

X is O or S;
n is 0 or 1;
R is H, Cl, F, Br, $CH_3$, $OCH_3$ or $OCH_2CH_3$;
$R_1'$ is $C_1$-$C_3$ alkyl, allyl or propargyl;
$R_2$ is $X_1R_8$ or OH;
$R_3$ is Cl, F, Br or $CF_3$;
$R_4$ is H, Cl, F or Br;
$R_5$ is $CF_3$;
$R_6$ is H or Cl;
$R_7$ is H, Cl, F, Br or $CF_3$;
$R_8$ is $C_1$-$C_4$ alkyl, benzyl, phenyl, $C_5$-$C_8$ cycloalkyl, $CH_2CH_2OCH_3$, $CH_2CH_2OCH_2CH_3$, $C_3$-$C_4$ alkenyl, $C_3$-$C_4$ alkynyl, $C_3$-$C_4$ alkenyl substituted with Cl, $CH_2CH_2OCH_2CH_2OCH_3$, $CH_2CH_2OCH_2CH_2OCH_2CH_3$, $CH_2CO_2CH_3$ or $CH(CH_3)CO_2CH_3$;
$X_1$ is O or S;

provided that for compounds of Formula II
when A is A-2 or A-6, then R is H;
and their agriculturally suitable salts.

2. Compounds of Claim 1 wherein $R_1'$ is $CH_3$ and $R_2$ is $X_1R_8$.

3. Compounds of Claim 2 wherein A is A-1, A-2, A-4 or A-5; R is H, Cl, $OCH_3$ or F; and $R_8$ is $C_1$-$C_4$ alkyl.

4. Compounds of Claim 3 wherein A is A-1.
5. Compounds of Claim 3 wherein A is A-2.
6. Compounds of Claim 3 wherein A is A-4.
7. Compounds of Claim 3 wherein A is A-5.
8. Compounds of Claim 3 wherein A is A-1 and $R_8$ is $C_1$-$C_2$ alkyl.
9. Compounds of Claim 3 wherein A is A-2 and $R_8$ is $C_1$-$C_2$ alkyl.
10. Compounds of Claim 3 wherein A is A-4 and $R_8$ is $C_1$-$C_2$ alkyl.
11. Compounds of Claim 3 wherein A is A-5 and $R_8$ is $C_1$-$C_2$ alkyl.
12. The compound of Claim 1 which is 3-chloro-4-(6-chloro-2-quinoxalinyloxy)-α-methylbenzeneacetic acid, methyl ester.
13. The compound of Claim 1, which is 4-(3-chloro-5-(trifluoromethyl)-2-pyridinyl oxy)-α-methylbenzeneacetic acid, methyl ester.
14. An agriculturally suitable composition for controlling the growth of undesired vegetation comprising an effective amount of a compound of Claim 1 and at least one of the following: surfactant, solid inert diluent, liquid inert diluent and mixtures of the foregoing.
15. An agriculturally suitable composition for controlling the growth of undesired vegetation comprising an effective amount of a compound of Claim 2 and at least one of the following: surfactant, solid inert diluent, liquid inert diluent and mixtures of the foregoing.
16. An agriculturally suitable composition for controlling the growth of undesired vegetation comprising an effective amount of a compound of Claim 3 and at least one of the following: surfactant, solid inert diluent, liquid inert diluent and mixtures of the foregoing.
17. An agriculturally suitable composition for controlling the growth of undesired vegetation comprising an effective amount of a compound of Claim 4 and at least one of the following: surfactant, solid inert diluent, liquid inert diluent and mixtures of the foregoing.
18. An agriculturally suitable composition for controlling the growth of undesired vegetation comprising an effective amount of a compound of Claim 5 and at least one of the following: surfactant, solid inert diluent, liquid inert diluent and mixtures of the foregoing.
19. An agriculturally suitable composition for controlling the growth of undesired vegetation comprising an effective amount of a compound of Claim 6 and at least one of the following: surfactant, solid inert diluent, liquid inert diluent and mixtures of the foregoing.
20. An agriculturally suitable composition for controlling the growth of undesired vegetation comprising an effective amount of a compound of Claim 7 and at least one of the following: surfactant, solid inert diluent, liquid inert diluent and mixtures of the foregoing.
21. An agriculturally suitable composition for controlling the growth of undesired vegetation comprising an effective amount of a compound of Claim 8 and at least one of the following: surfactant, solid inert diluent, liquid inert diluent and mixtures of the foregoing.
22. An agriculturally suitable composition for controlling the growth of undesired vegetation comprising an effective amount of a compound of Claim 9 and at least one of the following: surfactant, solid inert diluent, liquid inert diluent and mixtures of the foregoing.
23. An agriculturally suitable composition for controlling the growth of undesired vegetation comprising an effective amount of a compound of Claim 10 and at least one of the following: surfactant, solid inert diluent, liquid inert diluent and mixtures of the foregoing.
24. An agriculturally suitable composition for controlling the growth of undesired vegetation comprising an effective amount of a compound of Claim 11 and at least one of the following: surfactant, solid inert diluent, liquid inert diluent and mixtures of the foregoing.
25. An agriculturally suitable composition for controlling the growth of undesired vegetation comprising an effective amount of a compound of Claim 12 and at least one of the following: surfactant, solid inert diluent, liquid inert diluent and mixtures of the foregoing.
26. An agriculturally suitable composition for controlling the growth of undesired vegetation comprising an effective amount of a compound of Claim 13 and at least one of the following: surfactant, solid inert diluent, liquid inert diluent and mixtures of the foregoing.
27. A method for controlling the growth of undesired vegetation which comprises applying to the locus to be protected an effective amount of a compound of Claim 1.
28. A method for controlling the growth of undesired vegetation which comprises applying to the locus to be protected an effective amount of a compound of Claim 2.
29. A method for controlling the growth of undesired vegetation which comprises applying to the locus to be protected an effective amount of a compound of Claim 3.
30. A method for controlling the growth of undesired vegetation which comprises applying to the locus to be protected an effective amount of a compound of Claim 4.
31. A method for controlling the growth of undesired vegetation which comprises applying to the locus to be protected an effective amount of a compound of Claim 5.
32. A method for controlling the growth of undesired vegetation which comprises applying to the locus to be protected an effective amount of a compound of Claim 6.
33. A method for controlling the growth of undesired vegetation which comprises applying to the locus to be protected an effective amount of a compound of Claim 7.

34. A method for controlling the growth of undesired vegetation which comprises applying to the locus to be protected an effective amount of a compound of Claim 8.
35. A method for controlling the growth of undesired vegetation which comprises applying to the locus to be protected an effective amount of a compound of Claim 9.
36. A method for controlling the growth of undesired vegetation which comprises applying to the locus to be protected an effective amount of a compound of Claim 10.
37. A method for controlling the growth of undesired vegetation which comprises applying to the locus to be protected an effective amount of a compound of Claim 11.
38. A method for controlling the growth of undesired vegetation which comprises applying to the locus to be protected an effective amount of a compound of Claim 12.
39. A method for controlling the growth of undesired vegetation which comprises applying to the locus to be protected an effective amount of a compound of Claim 13.